# EUROPEAN PATENT SPECIFICATION

(11) **EP 3 549 565 B1**
(45) Date of publication and mention of the grant of the patent: **09.11.2022**
(21) Application number: 16926030.4
(22) Date of filing: 28.12.2016
(51) Int. Cl.: A61F 13/49, A61F 13/56, A61F 13/00

(54) **ABSORBENT ARTICLE**
ABSORBIERENDER ARTIKEL
ARTICLE ABSORBANT

(43) Date of publication of application: 09.10.2019
(73) Proprietor: Unicharm Corporation, Shikokuchuo-shi, Ehime 799-0111 (JP)
(72) Inventor: SAKAGUCHI, Satoru, Kanonji-shi Kagawa 769-1602 (JP); TAKAHASHI, Maika, Kanonji-shi Kagawa 769-1602 (JP)
(74) Representative: Dolleymores
(86) International application number: PCT/JP2016/089177
(87) International publication number: WO 2018/123046

(56) References cited:
- EP-A1- 2 250 982
- GB-A- 2 244 422
- JP-A- H11 503 032
- JP-A- 2014 064 627
- JP-A- 2016 029 969
- JP-B1- 6 073 539
- US-A1- 2005 027 279
- US-A1- 2005 043 699
- US-A1- 2007 191 796

## Description

### FIELD

The present invention relates to an absorbent article.

### BACKGROUND

Absorbent articles such as disposable diapers are known. Such absorbent articles include, for example, an absorbent body, and on the dorsal side of the absorbent body in the lengthwise direction, a pair of side flaps extending outward from both ends in the widthwise direction of the absorbent body. Patent Literature 1 discloses one such absorbent article, with side panels (side flaps) for a disposable product (absorbent article). In Patent Literature 1, an example is described wherein side flaps are formed by sandwiching a stretching member by two nonwoven fabrics that have been subjected to stretching treatment.

### CITATION LIST

### PATENT LITERATURE

PTL 1:Japanese Patent Public Inspection No. 11-506037
US 2007/0191796 A1 discloses an elasticized retaining panel for an absorbent garment, the panel including an outer web, an inner web and an elastic web disposed between the outer web and the inner web and bonded thereto. The elastic web comprises a network of reticulated strands.

### SUMMARY

### TECHNICAL PROBLEM

In Patent Literature 1, the side flaps are formed of a stretchable material, and therefore the absorbent article has improved compatibility with the body. To impart stretchability to the side flaps, however, it is necessary to also provide them with strength and rigidity to withstand tensile force when the side flaps have been pulled in the widthwise direction of the absorbent article. When an absorbent article having side flaps provided with strength and rigidity is put on by a wearer, the inner side edges in the lengthwise direction of the rigid side flaps contact relatively forcefully against or penetrate into the regions around the legs of the body, potentially causing the wearer to feel discomfort or lose a level comfort. In addition, the construction of the side flaps has been difficult to discern from the outside.

It is an object of the present invention to provide an absorbent article that has improved compatibility with the body while improving the fitted feel for the wearer and being more easily recognizable from the outside.

### SOLUTION TO PROBLEM

The present invention provides the absorbent article of independent claim 1. The dependent claims specify preferred but optional features.

The absorbent article of the present invention is as follows. (1) An absorbent article having a lengthwise direction, a widthwise direction and a thickness direction, and including an absorbent body, and a pair of side flaps which extend outward from both ends in the widthwise direction of the absorbent body, on a dorsal side in the lengthwise direction of the absorbent body, wherein each of the pair of side flaps includes: a stretching member which is in the form of a sheet and stretchable in the widthwise direction, and sheet members which are respectively layered on both sides in the thickness direction of the stretching member, and has: a stretching member placement section where the stretching member and the sheet members overlap in the thickness direction, and extending sections where the sheet members are extended respectively on an outer side and an inner side in the lengthwise direction of the stretching member placement section, wherein the sheet members are formed of nonwoven fabrics, at least a sheet member on a skin side, of the sheet members layered on both sides in the thickness direction of the stretching member, is a nonwoven fabric having a concavo-convex structure, the nonwoven fabric with the concavo-convex structure includes: a plurality of raised sections extending in the lengthwise direction and aligned at prescribed intervals in the widthwise direction, and a plurality of recess sections situated between mutually adjacent raised sections, and when the side flaps are in a state of their natural length, the extending sections have multiple wrinkles aligned in the widthwise direction, running along the lengthwise direction.

In this absorbent article, as mentioned above, an extending section, stretching member placement section and extending section are provided at the side flaps in that order from the outer side in the lengthwise direction, while in the thickness direction, two sheet members are layered at the extending sections, and a sheet member, a stretching member and a sheet member are provided at the stretching member placement section in that order from the top side.

Thus, in the stretching member placement section, the side flaps can be imparted with stretchability by the stretching member, and strength and rigidity can be imparted to the side flaps due to clamping from both sides by the sheet members. In other words, compatibility of the absorbent article with the body can be improved.

The extending sections are provided at the inner and outer edges in the lengthwise direction of the side flaps. When the wearer puts on the absorbent article, therefore, the inner side edges in the lengthwise direction of the side flaps, i.e. their extending sections, contact with the regions around the legs of the body. Since the extending sections are formed of nonwoven fabrics without including the stretching member, they have low strength and rigidity, and therefore the side flaps do not forcefully contact with the body, allowing a softer feel to be provided to the wearer. Furthermore, since the nonwoven fabrics have multiple wrinkles aligned in the widthwise direction, they easily stretch in approximately the widthwise direction, thereby allowing force acting on the body from the side flaps to be alleviated by elongation of the nonwoven fabrics even when the side flaps are able to forcefully contact with the body. This can therefore minimize discomfort experienced by the wearer, and can improve the level of comfort.

The wrinkles formed in the sheet members on the stretching member when the side flaps are in the state of their natural length are extremely small and barely noticeable to the user. Consequently, even though the stretching member is stretchable in the widthwise direction, the user may not realize that the stretching member is stretchable. In this absorbent article, however, the wrinkles formed in the sheet members at the extending sections where the stretching member is not present are larger than the wrinkles formed in the sheet members on the stretching member. These wrinkles are therefore more visible to the user. Thus, the user can discern from the outside that the side flaps are stretchable and that the large wrinkle folds appear to be soft.

It is thus possible to provide an absorbent article that has improved compatibility with the body while improving the fitted feel for the wearer and being more easily recognizable from the outside.

The absorbent article of the present invention may also be (2) the absorbent article according to (1) above, wherein at the extending section on the inner side in the lengthwise direction, a lengthwise inner side edge has, in a plan view, a wavy form where a portion curving in a convex manner inward in the lengthwise direction and a portion curving in a convex manner outward in the lengthwise direction, are alternately connected in the widthwise direction.

In this absorbent article, the portions including the inner side edges in the lengthwise direction of the extending sections which are on the inner sides in the lengthwise direction of the side flaps, contact with the regions around the legs of the body. The inner side edges of the extending sections of this absorbent article also have wavy forms in the plan view. Therefore, the portions including the wavy form edges contact with the regions around the legs of the body. In this case, the portions including the wavy form edges have greater area in contact with the body, compared to portions with conventional straight-line or gently curved edges. As a result, force acting on the body can be distributed to the edge-including portions, and thus pressure on the body can be reduced. This can minimize discomfort experienced by the wearer, and can improve the level of comfort. In addition, since the wavy form is easily visible to the user, it can be recognized from the outer side that the side flaps are stretchable, i.e. that the side flaps can be comfortably used without causing discomfort.

The absorbent article of the present invention may also be (3) the absorbent article according to (1) or (2) above, wherein the multiple wrinkles extend in the lengthwise direction from the extending sections so as to reach into the stretching member placement section.

Since the multiple wrinkles in this absorbent article run in the lengthwise direction from the extending sections reaching into the stretching member placement section, the multiple wrinkles are more easily visible to the user. It can therefore be more easily recognized from the outer side that the side flaps are stretchable, i.e. that the side flaps can be comfortably used without causing discomfort. Furthermore, since the multiple wrinkles run long from the extending sections reaching into the stretching member placement section, the nonwoven fabrics of the extending sections are more stretchable in approximately the widthwise direction. This allows force acting on the body from the side flaps to be alleviated by elongation of the nonwoven fabrics even when the side flaps are able to forcefully contact with the body.

The absorbent article of the present invention may also be (4) the absorbent article according to any one of (1) to (3) above, wherein the nonwoven fabric having the concavo-convex structure further includes: at bottom sections of the recess sections, a plurality of depressed sections formed intermittently along the lengthwise direction and extending in an opposite direction from a direction from the bottom sections of the recess sections toward the top sections of the raised sections, wherein the nonwoven fabric with the concavo-convex structure is layered on the stretching member so that the plurality of depressed sections are located on a side facing the stretching member.

When the nonwoven fabrics of the sheet members are formed by gear stretching, and the degree of stretching is increased in the attempt to obtain adequate stretchability, the nonwoven fabrics become partially torn. When this occurs, the stretching members become exposed from the torn sections of the nonwoven fabrics, potentially contacting directly with the skin of the wearer. In this absorbent article, however, the sheet members each have a three-layer structure of raised sections, recess sections and depressed sections. Thus, the degree of stretching of the raised sections and recess sections is about the same as the degree of stretching of the two-layer structure of the raised sections and recess sections, while the presence of the depressed sections as the third layer allows the stretchability of the nonwoven fabrics to be actually increased without tearing of the nonwoven fabrics. The sheet members can therefore be nonwoven fabrics with adequate stretchability, while it can also be skin-soft nonwoven fabrics with high flexibility. As a result, compatibility with the body can be increased while improving the fitted feel for the wearer. Incidentally, while the bottom sections of the depressed sections may be hardened by pressure, the bottom sections are disposed on the stretching member side so as to avoid contact with the skin, and therefore the hardness is not felt.

The absorbent article of the present invention may also be (5) the absorbent article according to (4) above, wherein the nonwoven fabrics of the sheet members on both a skin side and a non-skin side, of the sheet members layered on both sides in the thickness direction of the stretching member, are nonwoven fabrics having the concavo-convex structure.

In this absorbent article, the nonwoven fabrics of the sheet members on both sides of the stretching member are nonwoven fabrics having a concavo-convex structure. Thus, the nonwoven fabrics can be fabrics with adequate stretchability and also high flexibility, as well as skin softness not only for the wearer but also for a person fitting it (for example, a nurse or parent). Moreover, compatibility with the body can be further increased, while improving the fitted feel for the wearer and improving the usable feel and feeling of assurance for persons that are fitting it.

The absorbent article of the present invention may also be (6) the absorbent article according to any one of (1) to (5) above, wherein in the widthwise direction, an inner side edge of the stretching member is located further inward than an edge of the absorbent body.

In this absorbent article, since the stretching region of the stretching member is pulled inside the absorbent body in the widthwise direction, the distance between the inner side edges of the pair of side flaps can be shortened. This allows tensile force to be more easily transmitted to the absorbent body through the pair of side flaps, when the pair of side flaps have been pulled to both outer sides in the widthwise direction. It is thus possible to improve the fitted feel of the waist section. Compatibility with the body can therefore be further improved.

The absorbent article of the present invention may also be (7) the absorbent article according to any one of (1) to (6) above, wherein the extending section on the inner side in the lengthwise direction extends from an inner side edge to an outer side in the lengthwise direction, and has a plurality of cuts or weakening lines aligned intermittently in the widthwise direction.

An absorbent article must have a wide range of weight suitability and be able to cover various types of body frames, even with a single size. In this absorbent article, therefore, a plurality of cuts or weakening lines are formed at the inner side edges in the lengthwise direction of the extending sections, i.e. the regions around the legs of the side flaps. Thus, even with differences in body weight, the cuts or weakening lines open by movement of the leg-surrounding regions while the article is worn, so that the sizes of the openings of the leg-surrounding regions are altered, making it possible to reduce forceful contact or penetration of the side flaps into the regions around the legs, helping to minimize discomfort experienced by the wearer, and helping to improve the level of comfort. Here, the range within which the cuts or weakening lines open reaches no further than the stretching member placement section, due to the rigidity of the stretching members, and since the stretching member placement section does not tear, there is no effect on compatibility with the body. Furthermore, since the inner side edges in the lengthwise direction of the extending sections have multiple wrinkles, then even if a plurality of cuts or weakening lines are present they become diffused among the multiple wrinkles and are not recognized by the user, so that the user does not feel unnecessary anxiety.

The tear strength of the cuts is preferably from 0.05 N to 2 N. If the tear strength is too low then unnecessary tearing may take place, while if it is too high then tearing will become too difficult, and both situations may prevent the original function from being exhibited.

The absorbent article of the present invention may also be (8) the absorbent article according to any one of (1) to (7) above, wherein the sheet members extend further outward than outer side edges of the stretching member in the widthwise direction, and include outward extending sections without the multiple wrinkles, and each of the pair of side flaps further includes an engaging member which is connected to an outer end of the stretching member in the widthwise direction and to the outward extending section.

In this absorbent article, the outward extending sections of the sheet members that extend further outward than the outer side edges of the stretching members in the widthwise direction do not have multiple wrinkles. Therefore, the outward extending sections undergo almost no stretching, or in other words, they can have relatively high rigidity. When the wearer puts on the absorbent article, therefore, the engaging members can be stably held, thereby allowing the engaging members to be attached at the proper inward locations. In other words, the side flaps can be situated at the proper locations to minimize forceful contact or penetration of the side flaps with the regions around the legs, to minimize discomfort experienced by the wearer and to improve the level of comfort.

### ADVANTAGEOUS EFFECTS OF INVENTION

According to the present invention, it is possible to provide an absorbent article that has improved compatibility with the body while improving the fitted feel for the wearer and being more easily recognizable from the outside.

### BRIEF DESCRIPTION OF DRAWINGS

FIG. 1 is a plan view showing a construction example of an absorbent article according to an embodiment.
FIG. 2 is a cross-sectional view showing a construction example of an absorbent article according to an embodiment.
FIG. 3 is a cross-sectional view showing a construction example of an absorbent article according to an embodiment.
FIG. 4 is a plan view showing a construction example of side flaps according to an embodiment.
FIG. 5 is a diagram illustrating a method of producing an absorbent article according to an embodiment.
FIG. 6 is a diagram illustrating a method of producing an absorbent article according to an embodiment.
FIG. 7 is a diagram illustrating a method of producing an absorbent article according to an embodiment.
FIG. 8 is a plan view showing another construction example of the side flaps according to an embodiment.
FIG. 9 is a diagram showing a construction example of sheets for side flaps according to an embodiment.
FIG. 10 is a cross-sectional view showing another construction example of an absorbent article according to an embodiment.

### DESCRIPTION OF EMBODIMENTS

An absorbent article according to an embodiment will now be explained, using a tape-like (open type) disposable diaper as an example. However, the types and uses of the absorbent article of the present invention are not limited to this example, and the present invention may also be applied to other absorbent articles without departing from the scope of the present invention.

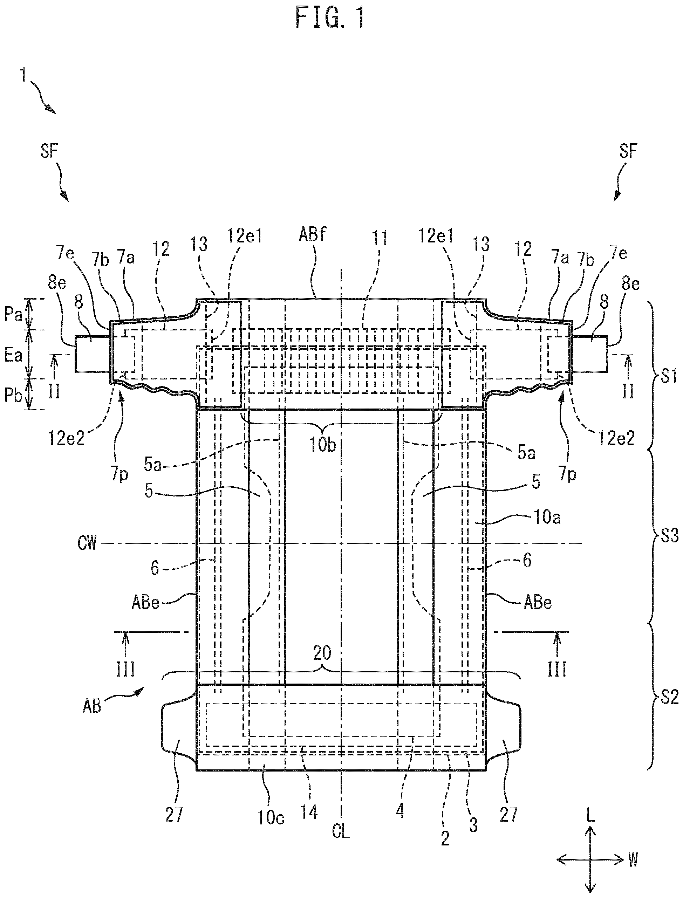

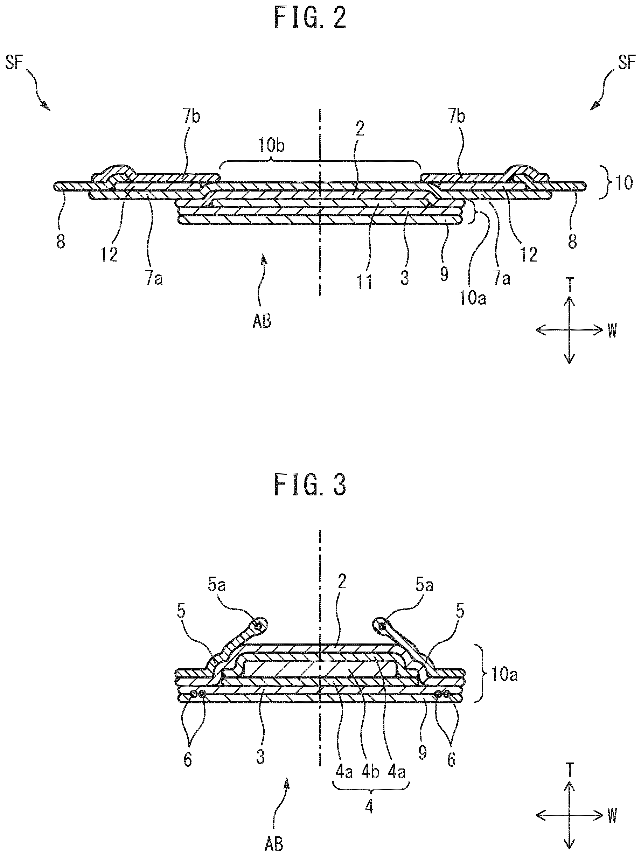

Figs. 1 to 3 are diagrams of an absorbent article 1 (disposable diaper). Fig. 1 is a plan view showing the state of the absorbent article 1 when spread out, Fig. 2 is a cross-sectional view along the line II-II of Fig. 1, and Fig. 3 is a cross-sectional view along the line III-III of Fig. 1. The absorbent article 1 has a lengthwise direction L, a widthwise direction W and a thickness direction T that are mutually perpendicular, and also has a lengthwise center axis line CL running through the center of the widthwise direction W and extending in the lengthwise direction L, and a widthwise center axis line CW running through the center of the lengthwise direction L and extending in the widthwise direction W. Also, the term "plan view" refers to viewing the deployed absorbent article 1 that has been spread out flat, from the upper side in the thickness direction. The terms "skin side" and "non-skin side" refer, respectively, to the side relatively near the side of the wearer's skin and the side relatively away from the side of the wearer's skin, in the thickness direction T of the absorbent article 1 when the absorbent article 1 is worn. The side nearer to and the side further from the lengthwise center axis line CL are the "inner side" and "outer side", respectively, in the widthwise direction W, and the direction toward and the direction away from it are the "inward" direction and the "outward" direction, respectively, in the widthwise direction W. The side nearer to and the side further from the widthwise center axis line CW are the "inner side" and "outer side", respectively, in the lengthwise direction L, and the direction toward and the direction away from it are the "inward" direction and the "outward" direction, respectively, in the lengthwise direction L. The term "in-plane direction" is an orientation that includes the lengthwise direction L and widthwise direction W. These definitions also apply to the materials and members that compose the absorbent article 1. Fig. 1 is a diagram of the absorbent article 1 as seen from the skin side.

The absorbent article 1 also has, in the lengthwise direction L, a dorsal side girth region S1 corresponding to the girth around the dorsal side of the wearer, an abdominal side girth region S2 corresponding to the girth around the abdominal side of the wearer, and a crotch region S3 located between the dorsal side girth region S1 and the abdominal side girth region S2, corresponding to the crotch region of the wearer. The dorsal side girth region S1, crotch region S3 and abdominal side girth region S2 are regions each constituting about 1/3 of the lengthwise direction L. The absorbent article 1 includes an absorbent body AB extending from the dorsal side girth region S1 to the abdominal side girth region S2 in the lengthwise direction L, a pair of side flaps SF, SF extending outward from both ends in the widthwise direction W of the absorbent body AB, in the dorsal side girth region S1. In the absorbent article 1, a pair of engaging members 8, 8 of the pair of side flaps SF, SF in the dorsal side girth region S1 engage with a target member 14 of the absorbent body AB in the abdominal side girth region S2, for fitting of the diaper. The crotch region S3 may also be narrowed inward in the widthwise direction W. The direction toward the dorsal side girth region S1 and abdominal side girth region S2 in the lengthwise direction L may also be referred to as the "dorsal side" and "abdominal side", respectively, or may be referred to as the "rear side" and "front side", respectively.

The absorbent body AB of the absorbent article 1 includes a front sheet 2 situated on the skin side, a back sheet 3 situated on the non-skin side, and an absorber 4 situated between the front sheet 2 and the back sheet 3. The front sheet 2 is a liquid-permeable sheet. The front sheet 2 may be any desired liquid-permeable sheet, such as, for example, a liquid-permeable nonwoven fabric or woven fabric, a liquid-permeable pore-formed synthetic resin film, or a composite sheet thereof. The back sheet 3 is a liquid-impermeable sheet. The back sheet 3 may be any desired liquid-impermeable sheet, such as, for example, a liquid-impermeable nonwoven fabric or synthetic resin film, a composite sheet thereof, an SMS nonwoven fabric, or the like. The absorber 4 is a fluid-absorbing and liquid-retaining material, and for this embodiment, it includes an absorbent body core 4b and core wraps 4a, 4a that subsume the absorbent body core 4b. The absorber 4 may be pulp fibers, synthetic fibers, an absorbing polymer or the like. The absorber 4 and each of the front sheet 2 and back sheet 3 are joined with an adhesive, and the front sheet 2 and back sheet 3 are joined by an adhesive at their peripheries. The adhesive used for joining between the front sheet 2, absorber 4 and back sheet 3 may be a known material that is commonly used in the absorbent article 1, such as a thermoplastic adhesive.

The absorbent body AB further includes a pair of anti-leakage walls 5, 5, a pair of leg stretching members 6, 6 and an outer sheet 9. The pair of anti-leakage walls 5, 5 cover the surfaces of both ends of the front sheet 2 in the widthwise direction W, while being formed on the inner side portions of the pair of side sheets extending along the lengthwise direction L. Each of the pair of anti-leakage walls 5, 5 has, on the surface of the end portion in the widthwise direction W of the front sheet 2, its fixed end at the outer side end in the widthwise direction W and its free end forming a stretchable gather at the inner side end in the widthwise direction W. Near each of the free ends, there are situated linear elastic solids 5a such as rubber, extending along the lengthwise direction L. The outer sheet 9 is disposed on the non-skin side of the back sheet 3 and reinforces the back sheet 3 while improving its hand feel. The pair of leg stretching members 6, 6 are linear elastic solids such as rubber extending along the lengthwise direction L on both sides in the widthwise direction W of the absorbent body AB in the crotch region S3, and they are situated between the outer sheet 9 and back sheet 3, for example, causing the portions contacting with the femoral region of the wearer to be stretched in the lengthwise direction L. A liquid-impermeable sheet is an example for the anti-leakage walls 5, and a hydrophobic, air-permeable sheet is an example for the outer sheet 9.

The absorbent body AB further includes a waist stretching member 11 in the dorsal side girth region S1. The waist stretching member 11 is a sheet-like member such as an elastomer having stretchability in the widthwise direction W, and it is disposed on the inner sides of the pair of side flaps SF, SF in the widthwise direction W and functions as a waist gather. The waist stretching member 11 is attached using an adhesive to any location of the surface on the skin side of the front sheet 2, the surface on the non-skin side of the back sheet 3, and a region between the front sheet 2 and the back sheet 3 in the dorsal side girth region S1. The waist stretching member 11 is disposed either partially overlapping, or not overlapping, with the absorber 4 in the plan view. For this embodiment, the waist stretching member 11 is disposed between the front sheet 2 and the back sheet 3 in such a manner that the portion of the waist stretching member 11 on the inner side in the lengthwise direction L overlaps with the absorber 4 in the thickness direction T. This can improve the fitting property of the absorbent article 1 in the girth region during wear.

Each of the pair of side flaps SF, SF includes, in the thickness direction T, a sheet-like stretching member 12 that is stretchable in the widthwise direction W, and a first sheet member 7a and second sheet member 7b respectively layered on both sides of the stretching member 12. The first sheet member 7a is layered on the surface of the non-skin side of the stretching member 12 while the second sheet member 7b is layered on the surface of the skin side of the stretching member 12. Furthermore, each of the side flaps SF has, in the lengthwise direction L, a stretching member placement section Ea where the first sheet member 7a and the second sheet member 7b overlap with the stretching member 12 in the thickness direction T, and extending sections Pa, Pb where the first and second sheet members 7a, 7b are extended to the outer side and inner side respectively of the stretching member placement section Ea. The stretching member 12 is not present in the extending sections Pa, Pb, however. That is, each side flap SF has, in order from the outer side to the inner side in the lengthwise direction L, the extending section Pa where double-layer sheet members 7a, 7b are layered, the stretching member placement section Ea where the stretching member 12 is situated between the double-layer sheet members 7a, 7b, and the extending section Pb where the double-layer sheet members 7a, 7b are layered. The ratio of the dimensions of the extending section Pa, stretching member placement section Ea and extending section Pb in the lengthwise direction L may be 1:1~4:1~3, for example.

In this absorbent article 1 having such a construction for the side flaps SF, stretchability can be imparted to each side flap SF by the stretching member 12 at the stretching member placement section Ea, and clamping from both sides through the first and second sheet members 7a, 7b helps to impart strength and rigidity to the side flap SF. That is, the side flaps SF can stretch without producing cracks, to deform along the body of the wearer, thus improving the body compatibility of the absorbent article 1.

For this embodiment, the absorbent body AB includes a body portion 10a including the front sheet 2, the absorber 4 and the back sheet 3, and a connecting sheet member 10b that is joined to the surface on the skin side of the dorsal side girth region S1 of the body portion 10a. The pair of side flaps SF, SF are also mutually connected and integrated by the connecting sheet member 10b. In other words, in the widthwise direction W, the inner side edge of one side flap SF of the pair of side flaps SF, SF and one of the edges of the connecting sheet member 10b are connected, while the inner side edge of the other side flap SF and the other of the edges of the connecting sheet member 10b are connected. For example, the inner side edge of the first sheet member 7a of one side flap SF and one of the edges of the connecting sheet member 10b are connected, while the inner side edge of the first sheet member 7a of the other side flap SF and the other of the edges of the connecting sheet member 10b are connected. For this embodiment in particular, the first sheet member 7a of the one side flap SF, the connecting sheet member 10b and the first sheet member 7a of the other side flap SF are integrated, being formed of a single sheet. This is preferred as it facilitates formation of the pair of side flaps SF, SF, and helps to equalize the distances of the pair of side flaps SF, SF from the widthwise center axis line CW in the lengthwise direction L, thereby facilitating production of the absorbent article 1. However, alternatively, the inner side edge of the second sheet member 7b of one side flap SF and one of the edges of the connecting sheet member 10b may be connected while the inner side edge of the second sheet member 7b of the other side flap SF and the other of the edges of the connecting sheet member 10b are connected. The connecting sheet member 10b may also be absent, in which case the pair of side flaps SF, SF will not be connected.

Known types of sheets may be used for the first and second sheet members 7a, 7b, and nonwoven fabrics are used for this embodiment. The basis weights of the first and second sheet members 7a, 7b are 5 g/m² to 100 g/m², for example. For this embodiment, the first and second sheet members 7a, 7b are each subjected to stretching treatment (for example, gear stretching treatment) before the stretching member 12 is layered and the side flaps SF are formed, so that they have the structure including recess sections and raised sections along the lengthwise direction L. The first and second sheet members 7a, 7b can thereby deform in response to stretching of the stretching member 12 in the widthwise direction W, without producing cracks or tears. In each of the first and second sheet members 7a, 7b, the interval between recess sections, i.e. the interval between raised sections, in the widthwise direction W, may be between 0.2 mm and 5 mm, for example. In the thickness direction T, the heights of the top sections of the raised sections, in reference to the bottom sections of the recess sections, are between 0.05 mm and 4 mm, for example. For this embodiment, the first sheet member 7a and the second sheet member 7b have approximately the same shape. The first sheet member 7a, stretching member 12 and second sheet member 7b are layered and joined together, while having both edges in the lengthwise direction L and the outer side edges in the widthwise direction W mutually overlapping, to form each of the side flaps SF. At each of the side flaps SF, the first sheet member 7a and second sheet member 7b are overlapping in the plan view. However, either the first sheet member 7a or the second sheet member 7b may be larger than the other.

The stretching member 12 may be a sheet of a known elastomer material, for example, a sheet of an elastomer resin such as natural rubber, synthetic rubber or rubber foam. For this embodiment, the stretching member 12 has an approximately rectangular shape that is somewhat smaller than the first and second sheet members 7a, 7b. At each of the side flaps SF, the stretching member 12 and the first and second sheet members 7a, 7b are joined so that the outer side edge 12e2 in the widthwise direction W of the stretching member 12 is separated in the widthwise direction W by a prescribed distance inward from the outer side edges 7e of the first and second sheet members 7a, 7b. Therefore, the first and second sheet members 7a, 7b, i.e. each of the pair of side flaps SF, SF, extend further outward than the outer side edge 12e2 in the widthwise direction W of the stretching member 12, and have outward extending sections 7p that do not include the stretching member 12.

For this embodiment, each side flap SF is subjected to stretching treatment (for example, gear stretching treatment), with the first sheet member 7a, stretching member 12 and second sheet member 7b in a layered state, thereby forming a stretching region 13 in the side flap SF which has recess sections and raised sections along the lengthwise direction L. The first sheet member 7a and second sheet member 7b of the side flap SF are more deformable in the widthwise direction W due to action of the stretching region 13, thereby allowing the first sheet member 7a, stretching member 12 and second sheet member 7b to be integrally more stretchable in the widthwise direction W at the side flap SF. However, the stretching region 13 does not overlap with the outward extending sections 7p or with the engaging member 8. Consequently, the bonding sections between the engaging member 8 and the other members do not suffer damage by stretching treatment. This allows adhesion between the engaging member 8 and the other members to be maintained, and can prevent tearing of the side flaps SF at the laminated sections that are subjected to more force when the engaging member 8 has been pulled.

For this embodiment, the inner side edge 12e1 of the stretching member 12 is situated further on the inner side than the edge ABe of the absorbent body AB, in the widthwise direction W. As a result, the distance between the inner side edges of the pair of side flaps SF, SF in the widthwise direction W can be shortened. This allows tensile force to be more easily transmitted to the absorbent body AB through the pair of side flaps SF, SF, when the pair of side flaps SF, SF have been pulled to both outer sides in the widthwise direction W. The fitted feel of the waist portion of the absorbent article 1 can thus be improved, and compatibility of the absorbent article 1 with the body can be improved. For this embodiment, the inner side edges of the first and second sheet members 7a, 7b are also situated further on the inner side than the edges ABe of the absorbent body AB, in the widthwise direction W. As a result, the strength of the inner side end of each of the pair of side flaps SF, SF in the widthwise direction W can be increased. This allows stable elongation without cracking or tearing at the inner side ends of the side flaps SF in the widthwise direction W, when it has been attempted to pull the pair of side flaps SF, SF to both outer sides in the widthwise direction W.

For this embodiment, each side flap SF further includes the engaging member 8 as mentioned above. The engaging member 8 is connected to the outer side edge of the stretching member 12 and the outward extending section 7p in the widthwise direction W, and extends from the outer side edge 7e in the widthwise direction W of the first and second sheet members 7a, 7b. The engaging member 8 is joined with an adhesive, for example, at one of a location between the first sheet member 7a and the second sheet member 7b in the thickness direction T, a location on the non-skin side of the first sheet member 7a and a location on the skin side of the second sheet member 7b. For this embodiment, it is disposed between the first sheet member 7a and the second sheet member 7b, further toward the skin side than the stretching member 12. Examples for the engaging member 8 include synthetic resin or nonwoven fabric sheet members, with hook-and-loop fasteners or adhesive tape being disposed in the region further outward in the widthwise direction W than the outward extending section 7p.

For this embodiment, the absorbent article 1 further includes, in the abdominal side girth region S2, a pair of abdominal side flaps 27, 27 that extend to both outer sides in the widthwise direction W of the body portion 10a of the absorbent body AB, an abdominal side connecting sheet 20 that is joined to the surface on the skin side of the body portion 10a and connects the pair of abdominal side flaps 27, 27 together, and a target member 14 disposed on the non-skin side of the body portion 10a. The pair of abdominal side flaps 27, 27 and the target member 14 are sheets where the engaging members 8, 8 are to be connected, the target member 14 being a loop of a hook-and-loop fastener when the engaging members 8, 8 are hooks of hook-and-loop fasteners, for example, while the abdominal side flaps 27 are nonwoven fabrics.

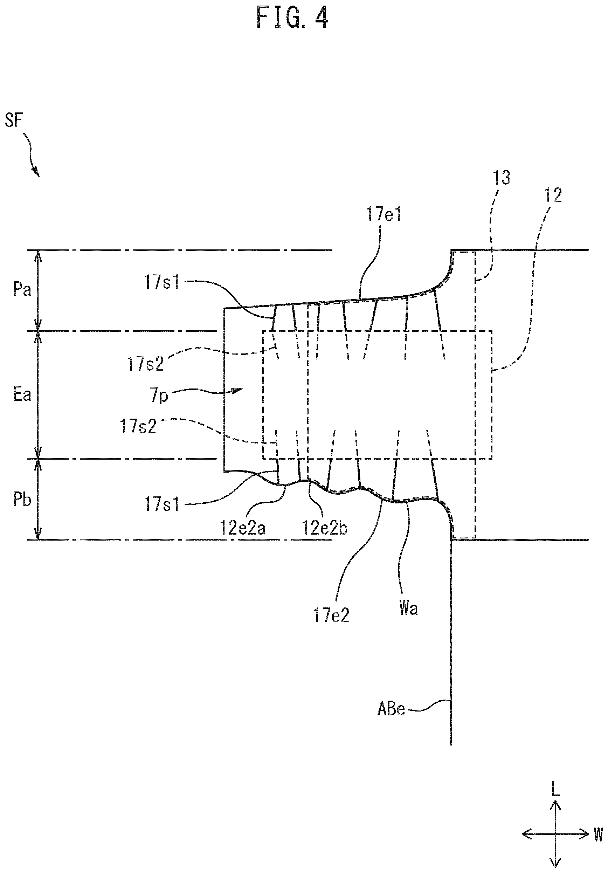

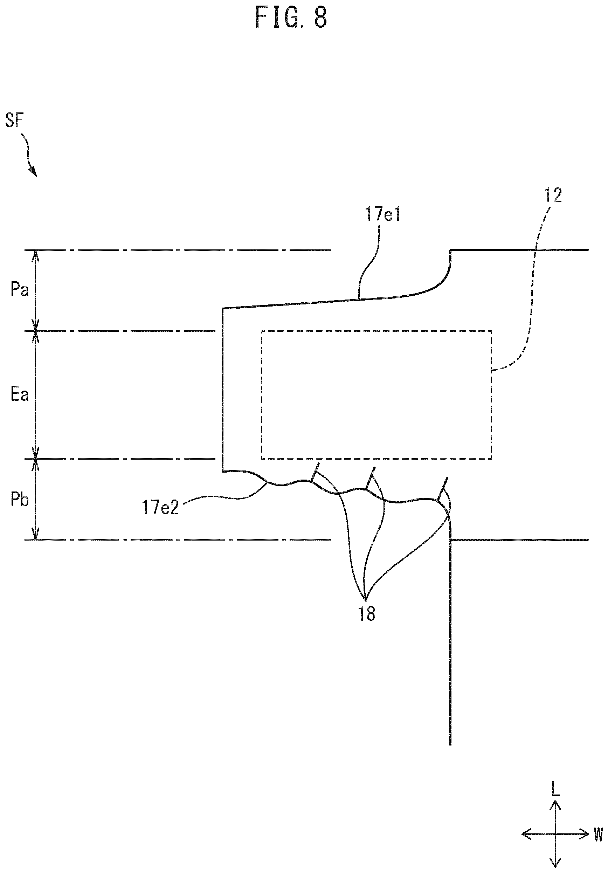

The side flaps SF will now be explained in detail. Fig. 4 is a magnified plan view of an example of the construction of one of the side flaps SF of Fig. 1. The engaging member 8 and stretching region 13 are omitted for simplicity (same for Fig. 8). In the side flap SF, at least the extending section Pa or Pb has multiple wrinkles 17s running along the lengthwise direction L and aligned in the widthwise direction W, when the side flap SF is in the state of its natural length. For this embodiment, the extending sections Pa, Pb have multiple wrinkles 17s1 running in the lengthwise direction L and aligned in the widthwise direction W. However, "wrinkles running in the lengthwise direction L" does not mean that the wrinkles must be completely parallel to the lengthwise direction L, and they may be inclined at angles of ±30° with respect to the lengthwise direction L. Also, "wrinkles aligned in the widthwise direction W" does not mean that the wrinkles must be aligned at equal intervals, and they may be partially overlapping or crossing, or branched. Moreover, so long as the wrinkles are present in the extending sections Pa, Pb running along the lengthwise direction L, it is not necessary for the extending sections to be completely traversing across the lengthwise direction L, nor is it necessary for them to start from the ends or terminate at the ends of the extending sections Pa, Pb in the lengthwise direction L. For example, when the side flaps SF are shapes that are nearly trapezoidal with upper bases of 5 cm, lower bases of 6 cm and heights of 5 cm, and the lower base is in contact with the absorbent body AB, the sizes of the wrinkles 17s1 may be such that their lengths in the lengthwise direction L are 5 mm or greater, for example, and no greater than the lengths of the extending sections Pa, Pb in the lengthwise direction L. The widths in the widthwise direction W may be 0.1 mm or greater, and within 2 mm, for example. The intervals in the widthwise direction W may be between 1 mm and 10 mm.

Such multiple wrinkles 17s1 can be formed by clamping the stretching member 12 by the first and second sheet members 7a, 7b. For example, the method may be that, when the side flaps SF are formed, the intervals or amplitudes of the recess sections and raised sections during stretching treatment of the first sheet member 7a and the intervals or amplitudes of the recess sections and raised sections during stretching treatment of the second sheet member 7b are different from each other. In this case, when both sheet members are to be joined by the extending sections Pa, Pb, wrinkles are formed in the extending sections Pa, Pb so that the intervals or amplitudes of the recess sections and raised sections do not match between both sheet members, forming an interference pattern by interference of waves with different periodicity. Since the stretching member 12 is joined to both sheet members at the stretching member placement section Ea, there is also an effect of the fact that the shapes of the recess sections and raised sections of both sheet members are unlikely to deform at the adjacent extending sections Pa, Pb. As another method, when the stretching member 12 is bonded to the first sheet member 7a during formation of the side flaps SF, the first sheet member 7a may be bonded while elongating the stretching member 12, for example. Since tension is applied to the first sheet member 7a immediately after bonding it does not undergo contraction in the cross-machine direction, but when the second sheet member 7b is subsequently joined and cut out into individual side flaps, and each stretching member 12 contracts to return to its natural length, and the recess sections and raised sections of the first and second sheet members 7a, 7b cannot match the fine wrinkles produced in the stretching member 12, so that wrinkles form in the extending sections Pa, Pb of the first and second sheet members 7a, 7b. Yet another method is to join both sheet members during formation of the side flaps SF, in a manner such that the direction in which the recess sections and raised sections extend in stretching treatment of the first sheet member 7a, and the direction in which the recess sections and raised sections extend in stretching treatment of the second sheet member 7b, are not parallel. As a result, crossing between the recess sections and raised sections of both sheet members at the extending sections Pa, Pb produces wrinkles, forming an interference pattern by interference of waves whose traveling directions do not match. In this case as well, since the stretching member 12 is joined to both sheet members at the stretching member placement section Ea, there is also an effect of the fact that the shapes of the recess sections and raised sections of both sheet members are unlikely to deform at the adjacent extending sections Pa, Pb. Each of these methods may be considered to be methods utilizing the concavo-convex structure of the first and second sheet members 7a, 7b formed by stretching treatment, and the multilayer structure of the first and second sheet members 7a, 7b and the stretching member 12 (including shape differences).

When a wearer puts on this absorbent article 1, the inner side edges of the side flaps SF in the lengthwise direction L, i.e. their extending sections Pb, contact with the regions around the legs of the body. Since the extending sections Pb are formed of nonwoven fabrics (the first and second sheet members 7a, 7b) without including the stretching member 12, they have low strength and rigidity and consequently the side flaps SF do not forcefully contact with the body, allowing a softer feel to be provided to the wearer. Furthermore, since the nonwoven fabrics have multiple wrinkles 17s1 aligned in the widthwise direction W due to stretching treatment, they easily stretch in approximately the widthwise direction W, thereby allowing force acting on the body from the side flaps SF to be alleviated by elongation of the nonwoven fabrics even when the side flaps SF are able to forcefully contact with the body. This can therefore minimize discomfort experienced by the wearer, and can improve the level of comfort.

When the side flaps SF are in the state of their natural length, the wrinkles formed in the first and second sheet members 7a, 7b of the stretching member placement section Ea are formed by restoration of the stretching member 12 to its natural length after having been attached to the first sheet member 7a in its elongated state, for example, but because they are extremely small they are barely visible to the user. Consequently, even though the stretching member 12 is stretchable in the widthwise direction W, the user may not notice the stretching member 12. In this absorbent article, however, the wrinkles 17s1 that are formed in the first and second sheet members 7a, 7b of the extending sections Pa, Pb when the stretching member 12 has been restored to its natural length from an elongated state, are larger than the wrinkles formed in the first and second sheet members 7a, 7b of the stretching member placement section Ea. These wrinkles 17s1 are therefore easily visible to the user. Thus, the user can be discern from the outside that the side flaps SF are stretchable and that the large wrinkle 17s1 folds appear to be soft.

It is thus possible to provide an absorbent article that has improved compatibility with the body while improving the fitted feel for the wearer and being more easily recognizable from the outside.

According to a preferred mode of this embodiment, the inner side edge 17e2 in the lengthwise direction L at the extending section Pb on the inner side in the lengthwise direction L has, in the plan view, a wavy form Wa where portions 17e2a curving in a convex manner inward in the lengthwise direction L and portions 17e2b curving in a convex manner outward in the lengthwise direction L are alternately connected in the widthwise direction W. Thus, in this absorbent article 1, the inner side edges 17e2 of the extending sections Pb that contact with the regions around the legs of the body have wavy forms Wa in the plan view. It is therefore possible to increase the area contacting with the body at the portions that include the edges 17e2. As a result, force acting on the body can be diffused by portions including the edges 17e2, and thus pressure on the body can be reduced. This can minimize discomfort experienced by the wearer, and can improve the level of comfort. In addition, since the wavy forms Wa are easily visible to the user, it can be recognized from the outer side that the side flaps are stretchable, i.e. that the side flaps can be comfortably used without causing discomfort.

According to a preferred mode of this embodiment, the multiple wrinkles 17s also include, in addition to the multiple wrinkles 17s1 extending in the lengthwise direction L through the extending sections Pa, Pb, also multiple wrinkles 17s2 extending in the lengthwise direction L from the extending sections Pa, Pb through the stretching member placement section Ea. In other words, the multiple wrinkles 17s extend in the lengthwise direction L from the extending sections Pa, Pb so as to reach into the stretching member placement section Ea. Since the multiple wrinkles 17s2 thus extend in the lengthwise direction L from the extending sections Pa, Pb reaching into the stretching member placement section Ea, the multiple wrinkles 17s1 + 17s2 are more easily visible to the user. It can therefore be more easily recognized from the outside that the side flaps SF are stretchable, i.e. that the side flaps can be comfortably used without causing discomfort. Furthermore, since the multiple wrinkles 17s1 + 17s2 extend long from the extending sections Pa, Pb reaching into the stretching member placement section Ea, the nonwoven fabrics (the first and second sheet members 7a, 7b) of the extending sections Pa, Pb are more easily stretchable in approximately the widthwise direction W. This allows force acting on the body from the side flaps SF to be alleviated by elongation of the nonwoven fabrics even when the side flaps SF are able to forcefully contact with the body.

In the absorbent article 1 according to a preferred mode of this embodiment, the outward extending sections 7p of the first and second sheet members 7a, 7b that extend further outward than the outer side edge 12e2 of the stretching members 12 in the widthwise direction W do not have multiple wrinkles. Therefore, the outward extending sections 7p can be made without wrinkles and with virtually no stretching, or in other words, relatively hard. When the wearer puts on the absorbent article 1, therefore, the engaging members 8 can be stably held, thereby allowing the engaging members 8 to be attached at the proper inward locations. In other words, the side flaps can be situated at the proper locations to minimize forceful contact or penetration of the side flaps with the regions around the legs, to minimize discomfort experienced by the wearer and to improve the level of comfort. According to yet another preferred mode of this embodiment, the portions where the engaging members 8 and the first and second sheet members 7a, 7b and stretching members 12 are layered are not present in the stretching region 13, and therefore have no wrinkles. That is, since the layered portions are not subjected to stretching treatment, the bonding sections between the engaging members 8 and the other members do not suffer damage. This allows adhesion between the engaging members 8 and the other members to be increased, and can prevent tearing of the side flaps SF at the layered portions that are subjected to more force when the engaging members 8 have been pulled.

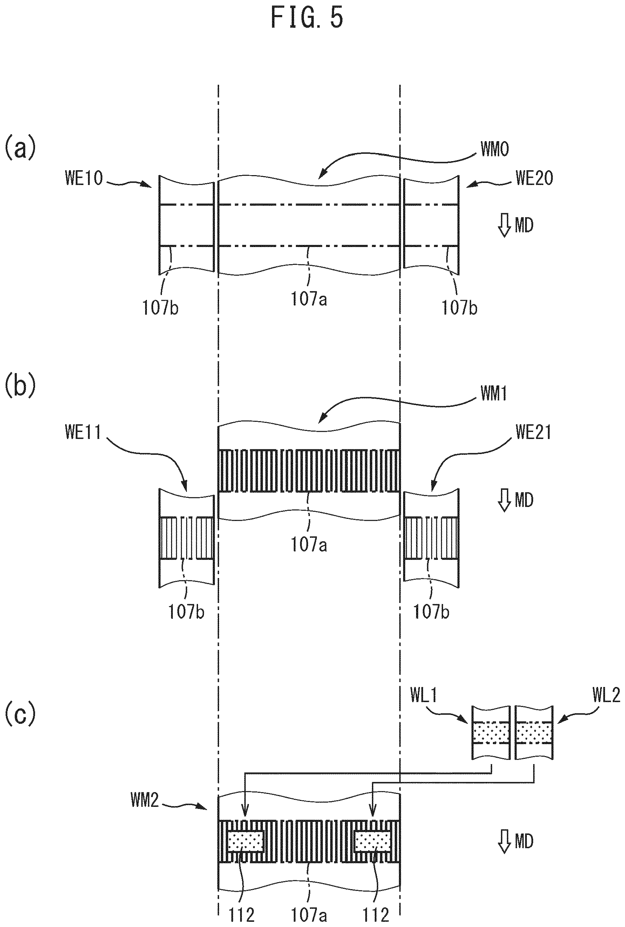

An example of a method of producing an absorbent article according to this embodiment will now be described. Fig. 5 to Fig. 7 schematically show an example of the construction of a sheet or semi-processed web, for explanation of the method of producing an absorbent article. Fig. 5 to Fig. 7 show each sheet or semi-processed web being transported along the machine direction (MD), from top to bottom in the diagrams, showing portions of each sheet or semi-processed web transporting continuously in the machine direction MD. This production method includes a cutting apart step, a side sheet stretching and joining step, a center sheet stretching step, a stretching member placement step, an engaging member joining step, a folding step, a semi-processed web stretching step, a shaping step and an absorbent body forming step. Incidentally, for this embodiment, the machine direction, the cross-machine direction (CD) perpendicular to the machine direction, and the thickness direction (TD) that is perpendicular to the machine direction and the cross-machine direction during the production process, are equivalent to the lengthwise direction L, widthwise direction W and thickness direction T, respectively, of the absorbent article 1 and its materials. Also, the direction toward and the direction away from an imaginary center axis line running in the machine direction through the center in the cross-machine direction of the transport surface are the inward and outward directions in the cross-machine direction.

First, in the cutting apart step, the material sheet in the form of a continuous sheet (not shown), is supplied to a cutting device (not shown) and is cut apart. Thus, as shown in Fig. 5(a), there are formed a center sheet WM0 and first and second side sheets WE10, WE20, adjacent to the one side and the other side, respectively, in the cross-machine direction of the center sheet WM0. The center sheet WM0 includes a portion 107a that includes the connecting sheet member 10b and the pair of first sheet members 7a, 7a. The first and second side sheets WE10, WE20 each include a portion 107b including a second sheet member 7b. Next, the center sheet WM0 is supplied to the center sheet stretching step, and the first and second side sheets WE10, WE20 are supplied to the side sheet stretching and joining step.

In the subsequent center sheet stretching step, the center sheet WM0 as a continuous sheet is supplied to a stretching apparatus (not shown), and stretching treatment (for example, gear stretching treatment) is carried out in the cross-machine direction. This forms a center sheet WM1 that is stretchable in the cross-machine direction, as shown in Fig. 5(b). The center sheet WM1 includes the portion 107a. The center sheet WM1 is supplied to the stretching member placement step.

In the side sheet stretching and joining step (first stage), the first and second side sheets WE10, WE20 as continuous sheets are supplied to a stretching apparatus (not shown), and stretching treatment (for example, gear stretching treatment) is carried out in the cross-machine direction. This forms first and second side sheets WE11, WE21 that are stretchable in the cross-machine direction, as shown in Fig. 5(b). The first and second side sheets WE11, WE21 include the portion 107b. The first and second side sheets WE11, WE21 are transported to a press roll (not shown). The side sheet stretching and joining step (second stage) will be explained later.

In the center sheet stretching step and the side sheet stretching and joining step (first stage), the shapes of the recess sections and raised sections formed in the sheet by the stretching treatment (the intervals between the raised sections or recess sections, the heights of the top sections of the raised sections compared to the bottom sections of the recess sections, and the angles formed by the directions of extension of the recess sections or raised sections with respect to the machine direction), may be the same or they may be different. The shapes of the recess sections and raised sections are set as appropriate based on the shapes of the wrinkles that are to be formed in the extending sections Pa, Pb.

In the subsequent stretching member placement step, a pair of stretching member sheets WL1, WL2 as continuous sheets are supplied to a cutting device (not shown), and are cut to prescribed dimensions in the machine direction. A pair of stretching members 112 are thus formed. The stretching members 112 are essentially the stretching members 12. Also, the sides of the pair of stretching members 112 that are coated with an adhesive are pressed against the center sheet WM1 with a press roll, thus attaching them. This causes the pair of stretching members 112 to be situated in a manner overlapping with the center sheet WM1 in the thickness direction, as shown in Fig. 5(c). The center sheet WM2 is thus formed. The center sheet WM2 is supplied to the engaging member joining step.

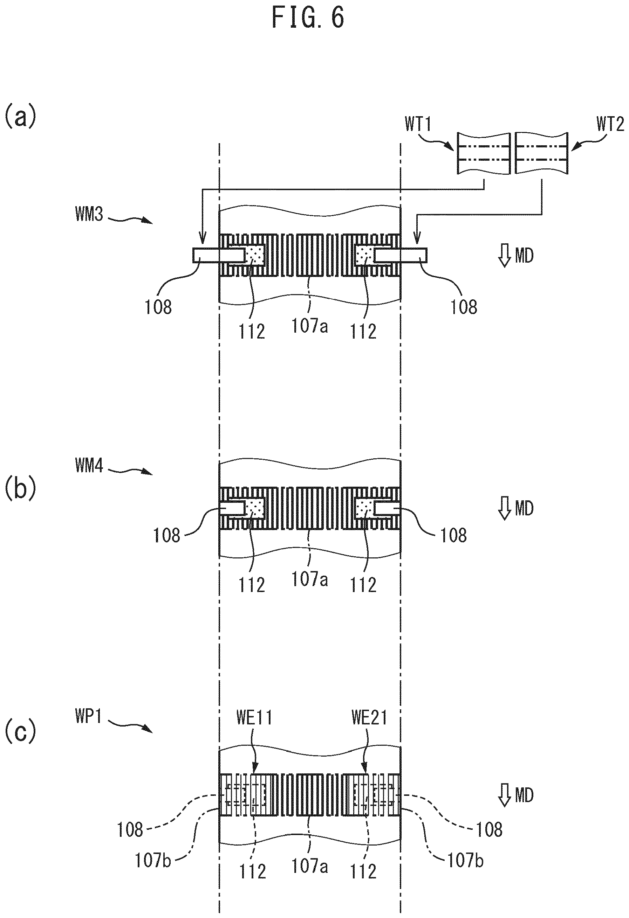

In the subsequent engaging member joining step, a pair of engaging member sheets WT1, WT2 as continuous sheets are supplied to a cutting device (not shown), and are cut to prescribed dimensions in the machine direction. A pair of engaging members 108 are thus formed. The engaging members 108 are essentially the engaging members 8. Also, the sides of the pair of engaging members 108 that are coated with an adhesive are pressed against the center sheet WM2 with a press roll, thus attaching them. This causes the pair of engaging members 108 to be situated in a manner overlapping with the pair of stretching members 112 and the center sheet WM2 in the thickness direction TD, as shown in Fig. 6(a). The center sheet WM3 is thus formed. The center sheet WM3 is supplied to the folding step.

In the subsequent folding step, as shown in Fig. 6(b), the extended sections of the pair of engaging members 108, 108 that extend from the locations of both edges of the center sheet WM3 in the cross-machine direction toward both outer sides are folded inward by a folding apparatus (not shown) in the cross-machine direction. In this case, folding is in a manner so as to cover the side of the center sheet WM3 opposite from the side on which the stretching members 112 are situated. The center sheet WM4 is thus formed. The center sheet WM4 is supplied to the latter process of the side sheet stretching and joining step.

Next, in the side sheet stretching and joining step (second stage), an adhesive (for example, a hot-melt adhesive) is coated onto one side of the first and second side sheets WE11, WE21 by a coating applicator (not shown). Press rolls press the surfaces of the first and second side sheets WE11, WE21 that have been coated with the adhesive, onto the stretching member 112 locations of the center sheet WM4. For this embodiment, in the cross-machine direction, the center sheet WM4 and the first side sheet WE11 and second side sheet WE21 are layered in such a manner that the outer side edge of the first side sheet WE11 and the outer side edge of the second side sheet WE21 overlap at both edges of the center sheet WM4. Thus, as shown in Fig. 6(c), a semi-processed sheet WP1 is formed having each of the first and second side sheets WE11, WE21 layered at both ends of the center sheet WM4. The semi-processed sheet WP1 is supplied to the semi-processed web stretching step.

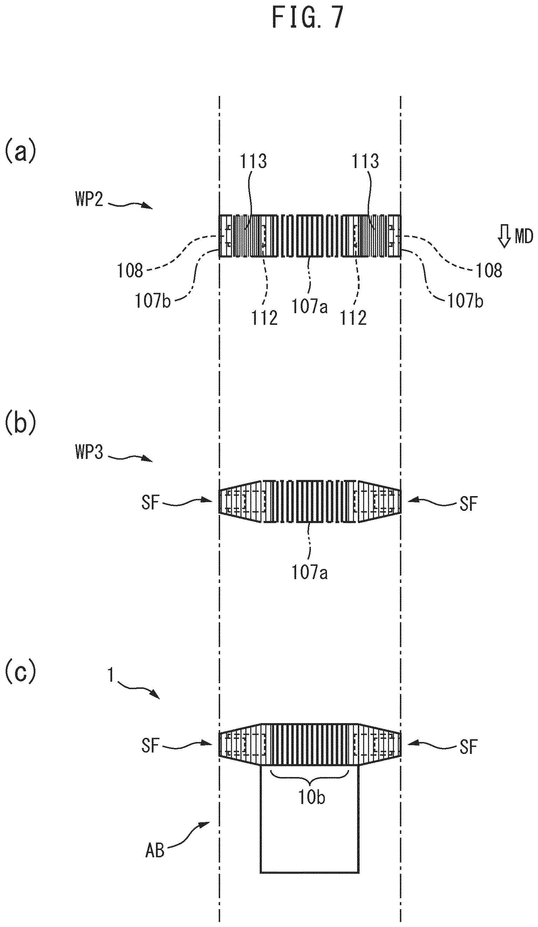

In the subsequent semi-processed web stretching step, the semi-processed sheet WP1 is supplied to a stretching apparatus (not shown). Then, as shown in Fig. 7(a), all or portions of both side sections of the semi-processed sheet WP1 (the locations where the first and second side sheets WE11, WE21 are layered) are subjected to stretching treatment (for example, gear stretching treatment) in the cross-machine direction, by a stretching apparatus. This forms a semi-processed sheet WP2 having stretchable stretching regions 113 formed in the cross-machine direction over all or portions of both ends of the semi-processed sheet WP1. The semi-processed sheet WP2 is supplied to the shaping step.

In the subsequent shaping step, the semi-processed sheet WP2 is supplied to a shaping apparatus (not shown). Then, as shown in Fig. 7(b), both side sections of the semi-processed sheet WP2 (the locations where the first and second side sheets WE11, WE21 are layered) are shaped into approximately trapezoidal shapes by the shaping apparatus. This forms a semi-processed sheet WP3 including a pair of side flaps SF, SF at both ends of the semi-processed sheet WP2. The semi-processed sheet WP3 is supplied to the remaining shaping step.

Next, the semi-processed sheet WP3 is cut off to prescribed dimensions in the machine direction by a cutting device (not shown), and each individual semi-processed sheet WP3 are formed, which is a pair of side flaps SF, SF connected by each individually separated connecting sheet member 10b. The pair of side flaps SF, SF connected by each individually separated connecting sheet member 10b are transported to the absorbent body forming step.

In the absorbent body forming step, as shown in Fig. 7(c), the pair of side flaps SF, SF connected by the connecting sheet member 10b are joined to the body portion 10a of a separately formed absorbent body AB. However, the pair of side flaps SF, SF connected by the connecting sheet member 10b may be joined to the body portion 10a with the skin side-facing surface and the non-skin side-facing surface reversed. The absorbent article 1 is produced by the process described above. Incidentally, this process is only an example, and the embodiment is not limited to this example.

Another preferred mode of this embodiment will now be explained. Fig. 8 is a plan view showing an example of another construction of the side flaps according to an embodiment. In this drawing, the multiple wrinkles 17s1, 17s2 are omitted for simplicity. According to another preferred mode of this embodiment, the extending section Pb on the inner side in the lengthwise direction L has a plurality of cuts or weakening lines 18 running from the inner side edge 17e2 in the lengthwise direction L toward the outer side in the lengthwise direction L, and aligned intermittently in the widthwise direction W. For this embodiment, however, the cuts are furrows where the first and second sheet members 7a, 7b of the extending section Pb have been cut in a linear fashion in the thickness direction. The cuts can be further widened by application of prescribed shear force. The weakening lines are linear portions where the thickness of the first and second sheet members 7a, 7b of the extending section Pb have been reduced from the original thickness. Upon application of a prescribed shear force, the weakening lines shear, forming cuts.

An absorbent article must have a wide range of body weight suitability and be able to cover various types of body frames, even with a single size. In this absorbent article 1, therefore, a plurality of cuts or weakening lines 18 are formed at the inner side edges of the extending sections Pb in the lengthwise direction L, i.e. the regions around the legs of the side flaps SF. Even with differences in body weight, therefore, opening of the cuts or weakening lines 18 by movement of the leg-surrounding regions while worn causes variation in the opening sizes of the leg-surrounding regions. This can minimize forceful contact or penetration of the side flaps SF with the regions around the legs, to minimize discomfort experienced by the wearer and to improve the level of comfort. In such cases, the range within which the cuts or weakening lines 18 open reaches no further than the stretching member placement section Ea, due to the rigidity of the stretching members 12, and since the stretching member placement section does not tear, there is no effect on compatibility with the body. Furthermore, since the inner side edges of the extending sections Pb in the lengthwise direction L have multiple wrinkles 17s1(, 17s2), then even if a plurality of cuts or weakening lines 18 are present they become diffused among the multiple wrinkles 17s1(, 17s2) and are not recognized by the user, so that the user does not feel unnecessary anxiety.

The sizes of the cuts or weakening lines are such that the lengths in the lengthwise direction L are 1 mm or greater, for example, and no greater than 1/2 of the lengths of the extending sections in the lengthwise direction L. The widths in the widthwise direction W may be 0.1 mm or greater, and within 2 mm, for example. The intervals in the widthwise direction W may be between 5 mm and 20 mm. The thicknesses of the weakening lines may be between 1/10 and 9/10 of the original thickness. The tear strength of the cuts or weakening lines is preferably from 0.05 N to 2 N. This is because if the tear strength is too low then unnecessary tearing may take place, while if it is too high then tearing will become too difficult, and both of these situations may prevent the original function from being exhibited.

### <Method of measuring tear strength>

The tear strength of a sheet member is the maximum load (tear strength) (N) measured when a sheet member in which cuts or weakening lines have been formed in the direction perpendicular to the tension direction is pulled in the tension direction by a tensile tester and has become ripped (torn), with "N/40 mm" meaning the tear strength (N) per 40 mm width. The tear strength can be measured according to the "Tear test" of JIS K 6772:1994, 7.5, for example. The specific procedure for the measurement is as follows. (a) A 40 mm width × 150 mm length sample piece is prepared, (b) A notch with a length of 75 mm is formed from the center of the short side of the sample piece, parallel to the long side. (c) The notched strip is held with a tensile tester and the tensile strength is measured at a pull rate of 200 mm/min. (d) The maximum strength measured is recorded as the tear strength (N/40 mm) of the sample.

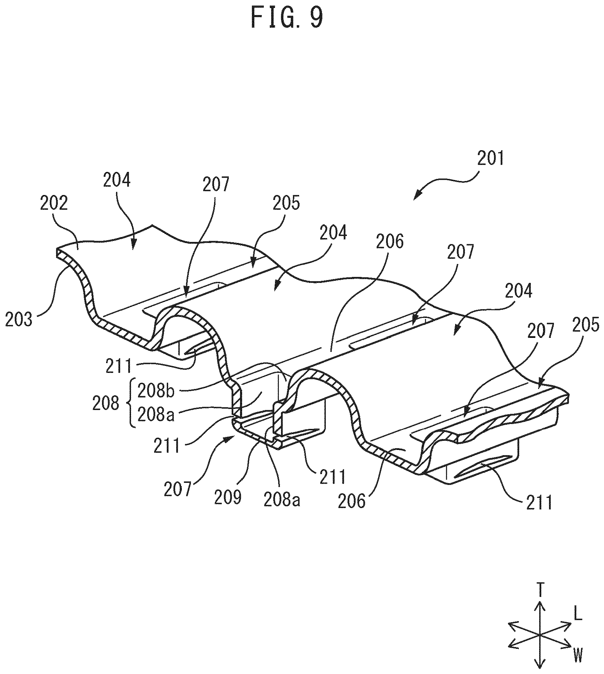

Another preferred mode of this embodiment will now be explained. Fig. 9 is a diagram showing an example of the construction of a sheet (nonwoven fabric) for a side flap according to an embodiment. The nonwoven fabric shown here is used for the first sheet member 7a.

The nonwoven fabric 201 has a first side 202 and a second side 203 on the side opposite the first side 202, and it includes a concavo-convex structure having raised sections 204 that are convex in the direction from the second side 203 toward the first side 202 and recess sections 205 that are concave in the direction from the first side 202 toward the second side 203. Of the first and second sheet members 7a, 7b layered on the stretching member 12, at least the first sheet member 7a on the skin side is a nonwoven fabric 201 having such a concavo-convex structure. Specifically, the nonwoven fabric 201 has a plurality of raised sections 204 extending in the lengthwise direction L and aligned at prescribed intervals in the widthwise direction W, a plurality of recess sections 205 situated between mutually adjacent raised sections 204, and a plurality of depressed sections 207 formed intermittently along the lengthwise direction L at the bottom sections 206 of the recess sections 205, and extending in the opposite direction from the direction toward the top sections of the raised sections 204. The nonwoven fabric 201 is layered on the stretching member 12 in such a manner that the plurality of depressed sections 207 are situated on the side facing (opposing) the stretching member 12.

When the nonwoven fabrics of the sheet member are formed by gear stretching, and the degree of stretching is increased in the attempt to obtain adequate stretchability, the nonwoven fabrics can potentially become partially torn. When this occurs, the stretching members become exposed from the torn sections of the nonwoven fabrics, potentially contacting directly with the skin of the wearer. In this absorbent article 1, however, the first sheet member 7a has a three-layer structure of the aforementioned raised sections 204, recess sections 205 and depressed sections 207. The first sheet member 7a can be formed by using the method described in Japanese Patent No. 5829326, for example, in the center sheet stretching step of the production method described above. By using the first sheet member 7a, the degree of stretching of the raised sections 204 and recess sections 205 can be the same as the degree of stretching of the two-layer structure comprising the raised sections 204 and recess sections 205, and therefore the spacings between the recess sections and between the raised sections, and the heights of the top sections of the raised sections with respect to the bottom sections of the recess sections can also be made the same, while the presence of the depressed sections 207 of the third layer can essentially increase the stretchability of the nonwoven fabric without causing tearing of the nonwoven fabric. The first sheet member 7a may therefore be a nonwoven fabric with adequate stretchability, so that a skin-soft nonwoven fabric with high flexibility may be used. As a result, compatibility with the body can be increased while improving the fitted feel for the wearer. Incidentally, while the bottom sections 209 of the depressed sections 207 may be hardened by pressure, the bottom sections 209 are disposed on the stretching member 12 side so as to avoid contact with the skin, and therefore they do not feel hard to the wearer.

The depressed sections 207 are preferably continuous with the bottom sections 206, and include wall-like peripheral surfaces 208 extending in the direction from the first side 202 toward the second side 203, and bottom sections 209 of the peripheral surfaces 208. The depressed sections 207 preferably have a structure including rectangular planar shaped openings and cuboid spaces with the openings at the top, having the peripheral surfaces 208 and bottom sections 209. The peripheral surfaces 208 include a pair of first peripheral surfaces 208a, 208a extending along the lengthwise direction L and situated facing each other, and a pair of second peripheral surfaces 208b, 208b extending along the widthwise direction W and situated facing each other. Preferably, holes 211 are formed in the first peripheral surfaces 208a, penetrating the first peripheral surfaces 208a and connecting with the second side 203 of the nonwoven fabric 201. For this embodiment, one hole 211 is formed in each first peripheral surface 208a. Each hole 211 is formed at a location near the bottom section 209 of the first peripheral surface 208a. The second peripheral surfaces 208b do not have holes 211.

The nonwoven fabric 201 tends to contact with the skin in the order of first the raised sections 204 and then the bottom sections 206 of the recess sections 205, with the raised sections 204 having the highest flexibility. Therefore, the raised sections 204 are preferably given a greater opportunity to contact the skin than the bottom sections 206, so that the smaller contact area of the nonwoven fabric 201 with the skin results in a more flexible feel. By providing the depressed sections 207, therefore, fewer portions of the bottom sections 206 of the recess sections 205 contact the skin, thus reducing the opportunity and area of contact with the skin. Moreover, by providing the holes 211 only on the first peripheral surfaces 208a, the tensile force of the fibers of the recess sections 205 and the adjacent raised sections 204 in which the holes 211 are formed is released, helping to increase the degree of freedom of movement of the raised sections 204 as a whole or of the fibers forming the raised sections 204. This can increase the flexibility of the raised sections 204, and particularly the flexibility of the raised sections 204 in the thickness direction T of the nonwoven fabric 201, as well as the flexibility in the in-plane direction (especially the widthwise direction W) of the nonwoven fabric 201 when sliding against the skin, to ensure a smooth touch sensation. Conversely, by not providing holes 211 in the second peripheral surfaces 208b, the level difference due to the presence of the depressed sections 207 can be made less likely to catch on the skin when the skin slides in the lengthwise direction L of the nonwoven fabric 201, i.e. in the direction in which the raised sections 204 or recess sections 205 extend, thus ensuring smoothness in the lengthwise direction L of the nonwoven fabric 201.

Another preferred mode of this embodiment will now be explained. The nonwoven fabric for a side flap shown in Fig. 9 may be used not only for the first sheet member 7a but also for the second sheet member 7b. That is, for this absorbent article 1, the nonwoven fabric 201 shown in Fig. 9 may be used as a nonwoven fabric for the first and second sheet members 7a, 7b that are layered on both sides in the thickness direction T of the stretching member 12. In other words, by using the nonwoven fabric 201, the side flaps can be provided with more adequate stretchability and higher flexibility. Furthermore, a skin-soft nonwoven fabric can thus be provided not only on the sides of the side flaps SF where the torso of the wearer contacts, but also on the sides where a person (for example, a nurse or parent) fitting the absorbent article 1 on the wearer may contact. Moreover, compatibility with the body can be further increased, while improving the fitted feel for the wearer and improving the usable feel and feeling of assurance for persons that are fitting it on.

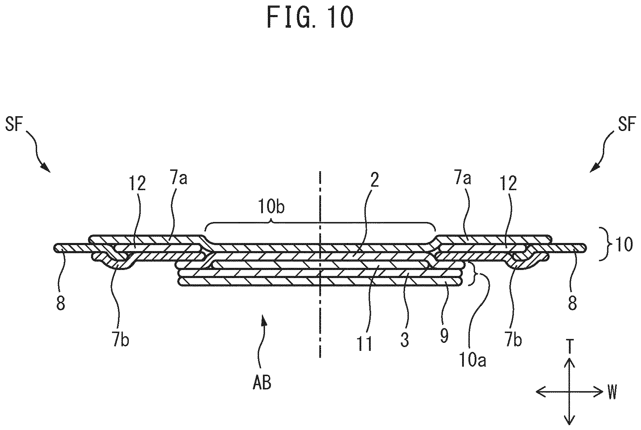

Another preferred mode of this embodiment will now be explained. Fig. 10 is a cross-sectional view showing another construction example of an absorbent article according to an embodiment. The structure in which the pair of side flaps SF, SF are mutually connected and integrated by the connecting sheet member 10b, i.e. the integrated structure of the side flaps, may also have the first sheet member 7a joined to the body portion 10a in a state facing the non-skin side, as shown in Fig. 2, or it may have the first sheet member 7a joined to the body portion 10a in a state facing the skin side, as shown in Fig. 10. In the absorbent body-forming step of this production method, an absorbent article 1 of this type can be produced by vertically inverting the side on which the structure wherein the side flaps are integrated is attached to the body portion 10a. In this case, the surface of the skin side of the structure wherein the side flaps are integrated consists of a single first sheet member 7a, and therefore a very skin-soft absorbent article 1 with no catching areas can be produced.

The absorbent article of the present invention is not limited to the embodiments described above and can incorporate appropriate combinations and modifications without departing from the object and scope of the present invention.

### REFERENCE SIGNS LIST

1 Absorbent article
7a, 7b Sheet member
12 Stretching member
17s Wrinkles
AB Absorbent body
Ea Stretching member placement section
Pa, Pb Extending section
SF Side flap

## Claims

1. An absorbent article (1) having a lengthwise direction (L), a widthwise direction (W) and a thickness direction (T), and comprising an absorbent body (AB), and a pair of side flaps (SF) which extend outward from both ends in the widthwise direction of the absorbent body (AB), on a dorsal side in the lengthwise direction of the absorbent body, wherein
each of the pair of side flaps (SF) comprises:
a stretching member (12) which is in the form of a sheet and stretchable in the widthwise direction (W) , and
sheet members (7a, 7b) which are respectively layered on both sides in the thickness direction of the stretching member (12),
and has:
a stretching member placement section (Ea) where the stretching member (12) and the sheet members (7a, 7b) overlap in the thickness direction (T), and
extending sections (Pa, Pb) where the sheet members (7a, 7b) are extended respectively on an outer side and an inner side in the lengthwise direction of the stretching member placement section (Ea), wherein
the sheet members (7a, 7b) are formed of nonwoven fabrics,
at least a sheet member (7a) on a skin side, of the sheet members layered on both sides in the thickness direction of the stretching member (12) , is a nonwoven fabric (201) having a concavo-convex structure,
the nonwoven fabric with the concavo-convex structure includes:
a plurality of raised sections extending in the lengthwise direction (L) and aligned at prescribed intervals in the widthwise direction (W), and
a plurality of recess sections situated between mutually adjacent raised sections, and
when the side flaps (SF) are in a state of their natural length, the extending sections (Pa, Pb) have multiple wrinkles (17s) aligned in the widthwise direction, running along the lengthwise direction (L).

2. The absorbent article (1) according to claim 1, wherein
at the extending section (Pb) on the inner side in the lengthwise direction, a lengthwise inner side edge (17e2) has, in a plan view, a wavy form (Wa) where a portion (17e2a) curving in a convex manner inward in the lengthwise direction and a portion (17e2b) curving in a convex manner outward in the lengthwise direction, are alternately connected in the widthwise direction (W).

3. The absorbent article (1) according to claim 1 or 2, wherein
the multiple wrinkles (17s) extend in the lengthwise direction (L) from the extending sections (Pa, Pb) so as to reach into the stretching member placement section (Ea).

4. The absorbent article (1) according to any one of claims 1 to 3, wherein
the nonwoven fabric (201) with the concavo-convex structure further includes:
at bottom sections of the recess sections (205), a plurality of depressed sections (207) formed intermittently along the lengthwise direction (L) and extending in an opposite direction from a direction from the bottom sections of the recess sections (205) toward top sections of the raised sections (204),
wherein the nonwoven fabric (201) with the concavo-convex structure is layered on the stretching member (12) so that the plurality of depressed sections (207) are located on a side facing the stretching member (12).

5. The absorbent article (1) according to any one of claims 1 to 4, wherein
the nonwoven fabrics of the sheet members (7a, 7b) on both a skin side and a non-skin side, of the sheet members (7a, 7b) layered on both sides in the thickness direction (T) of the stretching member (12), are nonwoven fabrics (201) having the concavo-convex structure.

6. The absorbent article (1) according to any one of claims 1 to 5, wherein
in the widthwise direction (W), an inner side edge (12e1) of the stretching member (12) is located further inward than an edge (ABe) of the absorbent body (AB).

7. The absorbent article (1) according to any one of claims 1 to 6, wherein
the extending section (Pb) on the inner side in the lengthwise direction (L) extends from an inner side edge (17e2) to an outer side in the lengthwise direction, and has a plurality of cuts or weakening lines (18) aligned intermittently in the widthwise direction (W).

8. The absorbent article (1) according to any one of claims 1 to 7, wherein
the sheet members (7a, 7b) extend further outward than outer side edges (12e2) of the stretching member (12) in the widthwise direction (W), and include outward extending sections (7p) without the multiple wrinkles (17s), and
each of the pair of side flaps (SF) further comprises an engaging member (8) which is connected to an outer end of the stretching member (12) in the widthwise direction (W) and to the outward extending section (7p).

## Patentansprüche

1. Absorbierender Artikel (1), der eine Längsrichtung (L), eine Breitenrichtung (W) und eine Dickenrichtung (T) aufweist und Folgendes umfasst: einen Saugkörper (AB) und ein Paar von Seitenklappen (SF), die sich nach außen von beiden Enden in der Breitenrichtung des Saugkörpers (AB) auf einer dorsalen Seite in der Längsrichtung des Saugkörpers erstrecken, wobei
jede des Paars von Seitenklappen (SF) Folgendes umfasst:
ein Dehnelement (12), das in der Form einer Lage vorliegt und in der Breitenrichtung (W) dehnbar ist, und
Lagenelemente (7a, 7b), die entsprechend auf beiden Seiten in der Dickenrichtung des Dehnelements (12) geschichtet sind,
und Folgendes aufweist:
einen Positionierungsabschnitt des Dehnelements (Ea), wo das Dehnelement (12) und die Lagenelemente (7a, 7b) in der Dickenrichtung (T) überlappen, und
sich erstreckende Abschnitte (Pa, Pb), wo sich die Lagenelemente (7a, 7b) entsprechend an einer Außenseite und einer Innenseite in der Längsrichtung des Positionierungsabschnitts des Dehnelements (Ea) erstrecken, wobei
die Lagenelemente (7a, 7b) aus Vliesstoffen ausgebildet sind,
mindestens ein Lagenelement (7a) auf einer Hautseite der Lagenelemente, die auf beiden Seiten in der Dickenrichtung des Dehnelements (12) geschichtet sind, ein Vliesstoff (201) ist, der eine konkave/konvexe Struktur aufweist,
der Vliesstoff mit der konkaven/konvexen Struktur Folgendes einschließt:
eine Vielzahl von erhobenen Abschnitten, die sich in der Längsrichtung (L) erstrecken und an vorgeschriebenen Abständen in der Breitenrichtung (W) ausgerichtet sind, und
eine Vielzahl von Aussparungsabschnitten, die sich zwischen beidseitig benachbarten, erhobenen Abschnitten befinden, und
wenn die Seitenklappen (SF) in einem Zustand ihrer natürlichen Länge vorliegen, die sich erstreckenden Abschnitte (Pa, Pb) mehrere Falten (17s) aufweisen, die in der Breitenrichtung ausgerichtet sind, entlang der Längsrichtung (L) verlaufen.

2. Absorbierender Artikel (1) nach Anspruch 1, wobei
an dem sich erstreckenden Abschnitt (Pb) auf der Innenseite in der Längsrichtung ein Längsinnenseitenrand (17e2) in einer Draufsicht eine wellige Form (Wa) aufweist, wo ein Teil (17e2a), der in einer konvexen Weise nach innen in der Längsrichtung gekrümmt ist, und ein Teil (17e2b), der in einer konvexen Weise nach außen in der Längsrichtung gekrümmt ist, in der Breitenrichtung (W) abwechselnd verbunden sind.

3. Absorbierender Artikel (1) nach Anspruch 1 oder 2, wobei
sich die mehreren Falten (17s) in der Längsrichtung (L) von den sich erstreckenden Abschnitten (Pa, Pb) derart erstrecken, um in den Positionierungsabschnitt des Dehnelements (Ea) hineinzureichen.

4. Absorbierender Artikel (1) nach einem der Ansprüche 1 bis 3, wobei
der Vliesstoff (201) mit der konkaven/konvexen Struktur weiter Folgendes einschließt:
an unteren Abschnitten der Aussparungsabschnitte (205) eine Vielzahl von vertieften Abschnitten (207), die intermittierend entlang der Längsrichtung (L) ausgebildet sind und sich in einer entgegengesetzten Richtung von einer Richtung von den unteren Abschnitten der Aussparungsabschnitte (205) zu oberen Abschnitten der erhobenen Abschnitte (204) erstrecken,
wobei der Vliesstoff (201) mit der konkaven/konvexe Struktur auf dem Dehnelement (12) derart geschichtet ist, dass sich die Vielzahl von vertieften Abschnitten (207) auf einer Seite befindet, die dem Dehnelement (12) zugewandt ist.

5. Absorbierender Artikel (1) nach einem der Ansprüche 1 bis 4, wobei
Die Vliesstoffe der Lagenelemente (7a, 7b) auf sowohl einer Hautseite als auch einer Nicht-Hautseite der Lagenelemente (7a, 7b), die auf beiden Seiten in der Dickenrichtung (T) des Dehnelements (12) geschichtet sind, Vliesstoffe (201) sind, die die konkave/konvexe Struktur aufweisen.

6. Absorbierender Artikel (1) nach einem der Ansprüche 1 bis 5, wobei
sich in der Breitenrichtung (W) ein Innenseitenrand (12e1) des Dehnelements (12) weiter nach innen befindet als ein Rand (ABe) des Saugkörpers (AB).

7. Absorbierender Artikel (1) nach einem der Ansprüche 1 bis 6, wobei
sich der erstreckende Abschnitt (Pb) auf der Innenseite in der Längsrichtung (L) von einem Innenseitenrand (17e2) zu einer Außenseite in der Längsrichtung erstreckt und eine Vielzahl von Einschnitten oder Schwächungslinien (18) aufweist, die intermittierend in der Breitenrichtung (W) ausgerichtet sind.

8. Absorbierender Artikel (1) nach einem der Ansprüche 1 bis 7, wobei
sich die Lagenelemente (7a, 7b) weiter nach außen erstrecken als Außenseitenränder (12e2) des Dehnelements (12) in der Breitenrichtung (W) und sich nach außen erstreckende Abschnitte (7p) ohne die mehreren Falten (17s) einschließen und
jede des Paars von Seitenklappen (SF) weiter ein eingreifendes Element (8) umfasst, das mit einem äußeren Ende des Dehnelements (12) in der Breitenrichtung (W) und mit dem sich nach außen erstreckenden Abschnitt (7p) verbunden ist.

## Revendications

1. Article absorbant (1) ayant une direction allant dans le sens de la longueur (L), une direction allant dans le sens de la largeur (W) et une direction allant dans le sens de l'épaisseur (T), et comportant un corps absorbant (AB), et une paire de rabats latéraux (SF) qui s'étendent vers l'extérieur en provenance des deux extrémités dans la direction allant dans le sens de la largeur du corps absorbant (AB), sur un côté dorsal dans la direction allant dans le sens de la longueur du corps absorbant, dans lequel
chacun de la paire de rabats latéraux (SF) comporte :
un élément extensible (12) qui est sous la forme d'une feuille et qui est en mesure de s'étirer dans la direction allant dans le sens de la largeur (W), et
des éléments formant feuille (7a, 7b) qui sont mis en couches respectivement des deux côtés dans la direction allant dans le sens de l'épaisseur de l'élément extensible (12),
et a :
une section de mise en place d'élément extensible (Ea) où l'élément extensible (12) et les éléments formant feuille (7a, 7b) se chevauchent dans la direction allant dans le sens de l'épaisseur (T), et
des sections d'extension (Pa, Pb) où les éléments formant feuille (7a, 7b) sont étendus respectivement sur un côté extérieur et un côté intérieur dans la direction allant dans le sens de la longueur de la section de mise en place d'élément extensible (Ea), dans lequel
les éléments formant feuille (7a, 7b) sont formés à partir de tissus non tissés,
au moins un élément formant feuille (7a) sur un côté peau, des éléments formant feuille mis en couches des deux côtés dans la direction allant dans le sens de l'épaisseur de l'élément extensible (12), est un tissu non tissé (201) ayant une structure concavo-convexe,
le tissu non tissé ayant la structure concavo-convexe comprend :
une pluralité de sections surélevées s'étendant dans la direction allant dans le sens de la longueur (L) et étant alignées selon des intervalles prescrits dans la direction allant dans le sens de la largeur (W), et
une pluralité de sections en retrait situées entre des sections surélevées mutuellement adjacentes, et
quand les rabats latéraux (SF) sont dans un état de leur longueur naturelle, les sections d'extension (Pa, Pb) ont de multiples plis (17s) alignés dans la direction allant dans le sens de la largeur, s'étendant le long de la direction allant dans le sens de la longueur (L).

2. Article absorbant (1) selon la revendication 1, dans lequel
au niveau de la section d'extension (Pb) sur le côté intérieur dans la direction allant dans le sens de la longueur, un bord latéral intérieur dans le sens de la longueur (17e2) a, dans une vue en plan, une forme ondulée (Wa) où une partie (17e2a) allant en se courbant d'une manière convexe vers l'intérieur dans la direction allant dans le sens de la longueur et une partie (17e2b) allant en se courbant d'une manière convexe vers l'extérieur dans la direction allant dans le sens de la longueur, sont raccordées tour à tour dans la direction allant dans le sens de la largeur (W).

3. Article absorbant (1) selon la revendication 1 ou la revendication 2, dans lequel
les multiples plis (17s) s'étendent dans la direction allant dans le sens de la longueur (L) en provenance des sections d'extension (Pa, Pb) de manière à atteindre jusque dans la section de mise en place d'élément extensible (Ea).

4. Article absorbant (1) selon l'une quelconque des revendications 1 à 3, dans lequel
le tissu non tissé (201) ayant la structure concavo-convexe comprend par ailleurs :
au niveau de sections inférieures des sections en retrait (205), une pluralité de sections en creux (207) formées de manière intermittente le long de la direction allant dans le sens de la longueur (L) et s'étendant dans une direction opposée par rapport à une direction en provenance des sections inférieures des sections en retrait (205) vers des sections supérieures des sections surélevées (204),
dans lequel le tissu non tissé (201) ayant la structure concavo-convexe est mis en couches sur l'élément extensible (12) de telle sorte que les sections en creux de la pluralité de sections en creux (207) sont situées sur un côté orienté vers l'élément extensible (12).

5. Article absorbant (1) selon l'une quelconque des revendications 1 à 4, dans lequel
les tissus non tissés des éléments formant feuille (7a, 7b) sur à la fois un côté peau et un côté non-peau, des éléments formant feuille (7a, 7b) mis en couches des deux côtés dans la direction allant dans le sens de l'épaisseur (T) de l'élément extensible (12), sont des tissus non tissés (201) ayant la structure concavo-convexe.

6. Article absorbant (1) selon l'une quelconque des revendications 1 à 5, dans lequel
dans la direction allant dans le sens de la largeur (W), un bord latéral intérieur (12e1) de l'élément extensible (12) est situé plus encore vers l'intérieur par rapport à un bord (ABe) du corps absorbant (AB).

7. Article absorbant (1) selon l'une quelconque des revendications 1 à 6, dans lequel
la section d'extension (Pb) sur le côté intérieur dans la direction allant dans le sens de la longueur (L) s'étend en provenance d'un bord latéral intérieur (17e2) jusqu'à un côté extérieur dans la direction allant dans le sens de la longueur, et a une pluralité de découpes ou de lignes d'affaiblissement (18) alignées de manière intermittente dans la direction allant dans le sens de la largeur (W).

8. Article absorbant (1) selon l'une quelconque des revendications 1 à 7, dans lequel
les éléments formant feuille (7a, 7b) s'étendent plus encore vers l'extérieur par rapport aux bords latéraux extérieurs (12e2) de l'élément extensible (12) dans la direction allant dans le sens de la largeur (W), et comprennent des sections s'étendant vers l'extérieur (7p) sans les multiples plis (17s), et
chacun de la paire de rabats latéraux (SF) comporte par ailleurs un élément de mise en prise (8) qui est raccordé à une extrémité extérieure de l'élément extensible (12) dans la direction allant dans le sens de la largeur (W) et à la section s'étendant vers l'extérieur (7p).
